# EUROPÄISCHE PATENTSCHRIFT

(11) **EP 0 639 562 B1**
(45) Veröffentlichungstag und Bekanntmachung des Hinweises auf die Patenterteilung: **29.01.1997**
(21) Anmeldenummer: 94112194.9
(22) Anmeldetag: 04.08.1994
(51) Int. Cl.: C07C 317/44, C07C 315/00, C07C 323/52

(54) **2-Cyanoethyl-2'-hydroxyethylsulfon, Verfahren zu seiner Herstellung und seine Verwendung zur Herstellung von 3-Aminopropyl-2'-hydroxyethylsulfon**
2-Cyanoethyl-2'-hydroxyethylsulphone, a method for preparing it and its use for the production of 3-aminopropyl-2'-hydroxyethylsulphone
Cyanoéthyl-2 hydroxyéthyl-2'-sulfone, sa préparation et son utilisation pour la préparation du aminopropyl-3 hydroxyéthyl-2'sulfone

(30) Priorität: 17.08.1993 DE 4327568
(43) Veröffentlichungstag der Anmeldung: 22.02.1995
(73) Patentinhaber: BAYER AG, 51368 Leverkusen (DE)
(72) Erfinder: Kunde, Klaus, Dr., D-53819 Neunkirchen-Seelscheid (DE)

(56) Entgegenhaltungen:
- EP-A- 0 518 321
- WO-A-91/13866
- WO-A-91/13867
- WO-A-92/15559
- CHEMICAL ABSTRACTS, Band 110, Nr. 13, 27. MUrz 1989, Columbus, Ohio, USA JAMES C.FISHBEIN et al. "Elimination reactions of beta-cyano thioethers: evidence for a carbanion intermediate and a change in rate-limiting step" Seite 615, Nr. 113 967d; & JP-A-61 005 075

## Beschreibung

3-Amino-propyl-2'-hydroxyethylsulfon ist eine geeignete Vorstufe zur Herstellung aliphatischer Vinylsulfone, die ihrerseits Zwischenstufen für die Herstellung von für Textilfarbstoffe geeigneten reaktiven Substituenten sind.

Es wurde nun gefunden, daß man ausgehend von 2-(2'-Cyanoethylmercapto)-ethanol

NC-CH₂CH₂-S-CH₂CH₂OH (I)

durch Oxidation zu 2-Cyanoethyl-2'-hydroxyethylsulfon

NC-CH₂CH₂-SO₂-CH₂CH₂OH (II)

und durch Reduktion der Nitrilgruppe zu 3-Amino-propyl-2'-hydroxyethylsulfon

H₂N-CH₂CH₂CH₂-SO₂-CH₂CH₂OH (III)

gelangen kann. Die Synthese von (III) aus (I) über die Zwischenstufe (II) stellt eine elegante Methode zur Herstellung von 3-Aminopropyl-2'-hydroxyethylsulfon dar.

Gegenstand der Erfindung ist also 2-Cyanoethyl-2'-hydroxyethylsulfon (II).

Die Verbindung (I) ist bekannt; sie kann z.B. durch Addition von 2-Mercaptoethanol an Acrylnitril hergestellt werden; J.H. Prager et al., J. Polym. Sci. Pt. A 2 (4) 1941-1953 (1964). Aus DE-A- 2 040 620 (= GB 1 354 906) und WO 91/13867 (= EP-A- 518 882) ist bereits bekannt, acylierte Aminoverbindungen, die eine Thioethergruppe enthalten, zu oxidieren. Gemäß Heinzmann, J. Am. Chem. Soc. 1982, 104, 6801 lassen sich Nitrile mit speziellen Borhydriden reduzieren.

Die Oxidation des Thioethers (I) kann durch Oxidationsmittel wie konzentrierte Salpetersäure, Kaliumpermanganat oder - vorzugsweise - Wasserstoffperoxid erfolgen. Als Reaktionsmedium wird vorzugsweise Wasser verwendet. pH-Werte von 2 bis 8, vorzugsweise von 5 bis 7 und Temperaturen von 0 bis 90°C werden bevorzugt. Es hat sich bewährt, die Oxidation bei niedrigerer Temperatur, z.B. bei 10 bis 30°C zu beginnen und die Temperatur im Laufe der Reaktion allmählich um 10 bis 20°C zu steigern, wobei das Oxidationsmittel entweder portionsweise oder kontinuierlich zugesetzt wird. Pro Mol Ausgangsprodukt werden im allgemeinen 2 bis 4, vorzugsweise 2 bis 2,5 Mol Oxidationsmittel eingesetzt. Der Fortgang der Reaktion kann beispielsweise dünnschichtchromatographisch verfolgt werden.

Es hat sich herausgestellt, daß die Oxidationsreaktion in Gegenwart katalytischer Wolframate oder Vanadate besonders glatt abläuft. Geeignete Wolframate und Vanadate umfassen insbesondere die Ammonium-, Kalium- und Natriumsalze. Mengen von 0,05 bis 5 g Wolframat bzw. Vanadat pro Mol Thioether (I) sind ausreichend; es genügt, wenn Wolframat bzw. Vanadat in einem fortgeschrittenen Stadium der Reaktion (beispielsweise ab einem Umsatz von 50 %) zugesetzt wird.

Weiterer Gegenstand der Erfindung ist also ein Verfahren zur Herstellung von 2-Cyanoethyl-2'-hydroxyethylsulfon (II) aus 2-(2'-Cyanoethylmercapto)-ethanol (I) durch Oxidation.

Obwohl Raney-Nickel ein besonders aktives Hydriermittel ist, hat es sich bei der Hydrierung der Nitrilgruppe des 2-Cyanoethyl-2'-hydroxyethylsulfons (II) nicht bewährt: trotz Drücken von bis zu 80 bar haben wir nur schlechte Ausbeuten erzielt.

Andererseits reichen für die Hydrierung von Nitrilgruppen die Boranate nach Lehrbuch nicht aus, sondern es muß das aktivere Lithiumaluminiumhydrid eingesetzt werden; vgl. Organikum, 10. Auflage, VEB Deutscher Verlag der Wissenschaften, Berlin 1971, S. 548.

Vor diesem Hintergrund ergab sich der folgende überraschende Befund:

Die Nitrilgruppe des 2-Cyanoethyl-2'-hydroxyethylsulfons (II) kann mittels Boranaten wie z.B. Lithiumborhydrid und - vorzugsweise - Natriumborhydrid in Mengen von 0,75 bis 3 Mol hydriert werden. Geeignete Reaktionstemperaturen sind 0 bis 50, vorzugsweise 10 bis 30°C. Die Hydrierung kann in einem unter Reaktionsbedingungen inerten organischen Lösungsmittel stattfinden. Bevorzugte organische Lösungsmittel sind C₁-C₄-Alkanole wie Methanol und Ethanol. Bevorzugtes Lösungsmittel ist Wasser.

Die Hydrierung erfolgt besonders vorteilhaft in Gegenwart von Cobalt- und/oder Nickelverbindungen. Geeignete Verbindungen umfassen Salze anorganischer oder organischer Säuren wie beispielsweise die Chloride, Fluoride, Bromide, Carbonate, basische Carbonate, Nitrate, Sulfate, Phosphate und Formiate, Acetate, Oxalate, Sulfamate, wie z.B. CoCl₂, CoCl₂ ^{·} 6H₂O, CoF₂, Co(NO₃)₂ ^{·} 6H₂O, Co(CH₃COO)₂ ^{·} 4H₂O, CoSO₄ ^{·} 7H₂O, NiCl₂, NiCl₂ ^{·} 6H₂O, NiBr₂, NiCO₃ ^{·} Ni(OH)₂ ^{·} 4H₂O, Ni(NO₃)₂ ^{·} 6H₂O, NiSO₄ ^{·} 6H₂O, Ni(CH₃COO)₂ ^{·} 4H₂O, Ni(HCOO)₂ ^{·} 2H₂O, Ni(COO)₂ ^{·} xH₂O, Ni(NH₂SO₃)₂ ^{·} xH₂O.

Die Co- bzw. Ni-Verbindungen werden im allgemeinen in Mengen von 0,01 bis 0,5 Mol pro Mol Sulfon (II) zugegeben.

Die Hydrierung (II) → (III) kann folgendermaßen durchgeführt werden: Das Sulfon (II) in einem organischen Lösungsmittel oder in Wasser wird nach Zugabe von Co- bzw. Ni-Salz mit Boranat in organischem Lösungsmittel oder Wasser versetzt. Man kann aber auch ein Co- bzw. Ni-Salz in Wasser lösen oder in organischem Lösungsmittel suspendieren und Boranat zusetzen; die erhaltene Suspension kann man dem Sulfon (II) zusetzen oder umgekehrt; falls nicht die Gesamtmenge des notwendigen Boranats von Anfang an zugegeben worden ist, kann man den Rest dann allmählich oder portionsweise zugeben. Nach Beendigung der Reaktion kann man den Niederschlag abtrennen und das Filtrat mit Säure, z.B. Salz- oder Schwefelsäure neutralisieren und bis zur beginnenden Trockne einengen.

Weiterer Gegenstand der Erfindung ist also die Verwendung des Sulfons (II) zur Herstellung des Amins (III) durch Hydrierung in Gegenwart von Boranat.

Es hat sich als überaus günstig erwiesen, das Amin (III) aus 2-(2'-Cyanoethylmercapto)-ethanol (I) über die Stufe des Sulfons (II) herzustellen.

Die Verwendung des 3-Aminopropyl-2'-hydroxyethylsulfons zur Herstellung von Textilfarbstoffen kann etwa analog EP-A 385 426 erfolgen.

Die Prozentangaben des nachfolgenden Beispiels beziehen sich auf das Gewicht.

### Beispiel

Zu einer Mischung von 53 g Acrylnitril und 1 g Piperidin werden bei 40°C 78 g 2-Mercaptoethanol zugetropft. Zur Vervollständigung der Reaktion wird noch eine Stunde bei dieser Temperatur gerührt.

Nach Zugabe von 300 ml Wasser und 0,5 g Natriumwolframatdihydrat (NaWO₄ + 2H₂O) werden bei 40°C langsam 68 g Wasserstoffperoxid (als 35 %ige wäßrige Lösung) zugetropft. Nach Beendigung der Oxidation erhält man eine waßrige Lösung von 2-(2'-Cyanoethylsulfonyl)ethanol welches nach Abdestillieren des Wassers als Öl erhalten werden kann; das ¹H-NMR-Spektrum (Lösemittel Dimethylsulfoxid-d₆) zeigt folgende Signale: δ = 2,97 t (2H), 3,31 t (2H), 3,5 t (2H), 3,82 m (2H), 5,24 t (1H).

Zu der wäßrigen Lösung des Oxidationsproduktes werden bei 20°C 9,5 g Cobaltchloridhexahydrat (CoCl₂ ^{·} 6H₂O) zugegeben. Die Lösung wird neutralisiert, dann werden bei 20°C in mehreren Portionen 57 g Natriumborhydrid zugesetzt. Nach Beendigung der Hydrierung wird das Cobaltborid abfiltriert; man erhält eine wäßrige Lösung von 3-Aminopropyl-2'-hydroxyethylsulfon.

Die Hydrierung gelingt ebenso gut, wenn man statt des Cobaltchloridhexahydrates eine äquimolare Menge Nickelchloridhexahydrat (NiCl₂ ^{·} 6H₂O) verwendet.

## Patentansprüche

1. Verbindung der Formel
NC-CH₂CH₂-SO₂-CH₂CH₂OH.

2. Verfahren zur Herstellung der Verbindung nach Anspruch 1 durch Oxidation von 2-(2'-Cyanoethylmercapto)-ethanol.

3. Verwendung der Verbindung nach Anspruch 1 zur Herstellung von 3-Aminopropyl-2'-hydroxyethylsulfon durch Hydrierung in Gegenwart von Boranat.

## Claims

1. Compound of the formula
NC-CH₂CH₂-SO₂-CH₂CH₂OH.

2. Process for preparing the compound according to Claim 1 by oxidation of 2-(2'-cyanoethylmercapto)-ethanol.

3. Use of the compound according to Claim 1 for preparing 3-aminopropyl 2'-hydroxyethyl sulphone by hydrogenation in the presence of boranate.

## Revendications

1. Composé de formule
NC-CH₂CH₂-SO₂-CH₂CH₂OH.

2. Procédé pour la préparation du composé selon la revendication 1 par oxydation de 2-(2'-cyanoéthylmercapto)-éthanol.

3. Utilisation du composé selon la revendication 1 pour la préparation de 3-aminopropyl-2'-hydroxyéthylsulfone par hydrogénation en présence de boranate.
